(19) Europäisches Patentamt
European Patent Office
Office européen des brevets

(11) Veröffentlichungsnummer: 0 011 150
B1

(12) # EUROPÄISCHE PATENTSCHRIFT

(45) Veröffentlichungstag der Patentschrift: 23.03.83

(51) Int. Cl.³: C 07 C 31/04, C 07 C 29/15, B 01 J 23/80

(21) Anmeldenummer: 79104018.1

(22) Anmeldetag: 18.10.79

(54) **Verfahren zur Herstellung von Methanol durch Hydrierung von Kohlenoxiden sowie Verfahren zur Herstellung eines Katalysators für diese Hydrierung.**

(30) Priorität: 26.10.78 DE 2846614

(43) Veröffentlichungstag der Anmeldung: 28.05.80 Patentblatt 80/11

(45) Bekanntmachung des Hinweises auf die Patenterteilung: 23.03.83 Patentblatt 83/12

(84) Benannte Vertragsstaaten: AT BE DE FR GB IT NL

(56) Entgegenhaltungen:
DE-A-1 568 864
DE-A-2 056 612
DE-A-2 132 020
US-A-3 923 694

(73) Patentinhaber: BASF Aktiengesellschaft, Carl-Bosch-Strasse 38, D-6700 Ludwigshafen (DE)

(72) Erfinder: Broecker, Franz Josef, Dr., Schwanthaler Allee 20, D-6700 Ludwigshafen (DE)
Erfinder: Gruendler, Karl-Heinz, Dr., Hockenheimer Strasse 16, D-6703 Limburgerhof (DE)
Erfinder: Marosi, Laszlo, Dr., Londoner Ring 11, D-6700 Ludwigshafen (DE)
Erfinder: Schwarzmann, Matthias, Dr., Carl-Bosch-Strasse 54, D-6703 Limburgerhof (DE)
Erfinder: Triebskorn, Bruno, Dr., Mohnstrasse 23, D-6700 Ludwigshafen (DE)
Erfinder: Zirker, Guenter, Dr., Schelmenzeile 78, D-6700 Ludwigshafen (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Verfahren zur Herstellung von Methanol durch Hydrierung von Kohlenoxiden sowie Verfahren zur Herstellung eines Katalysators für diese Hydrierung

Die Erfindung betrifft ein Verfahren zur Herstellung von Methanol durch Umsetzung von Wasserstoff, Kohlenmonoxid und Kohlendioxid und/oder Wasserdampf und/oder Sauerstoff in Gegenwart von Katalysatoren, die Zink, Kupfer und Aluminium enthalten, und ein Verfahren zur Herstellung eines Katalysators hierfür.

Es ist bekannt, Gemische von Kohlenmonoxid und Wasserstoff, gegebenenfalls unter Zusatz von Kohlendioxid, in Gegenwart von Katalysatoren bei Drücken zwischen 30 und 400 atü und bei Temperaturen zwischen 200 und 400°C zu Methanol umzusetzen. Als Katalysatoren werden meist Zinkoxid/Chromoxid- oder Kupferkatalysatoren verwendet, die noch zusätzliche Oxide enthalten können. Die bekannten Verfahren erfüllen noch nicht alle Anforderungen, bezüglich Wirtschaftlichkeit, Ausbeute an Endstoff, Einfachheit der Arbeitsweise, Raumzeitausbeute, Aktivität und Lebensdauer des Katalysators die an ein großtechnisches Verfahren gestellt werden.

Aus DE-A-2 056 612 ist bereits eine Mischkristallverbindung aus Cu, Zn und Al vom Mannaseittyp als Katalysatorvorläufer bekannt, bei dem die Aluminiummenge 25% beträgt, einen vergleichsweise hohen Cu-Gehalt aufweist und in den Raumzeitausbeuten nicht befriedigt. Auch aus DE-A-2 132 020 ist ein Mischkristallkatalysator bekannt. Dieser Katalysator wird aber für die Oxidation des Kohlenmonoxids bei der Entgiftung von Motorabgasen verwendet.

Die Erfindung geht aus von einem Verfahren zur Herstellung von Methanol durch Umsetzung eines Gasgemisches, enthaltend Kohlenmonoxid und Wasserstoff, bei Temperaturen von 200 bis 350°C und bei Drücken im Bereich von 50 bis 250 bar in Gegenwart eines Katalysators, der Zink, Kupfer und Aluminium enthält.

Die Erfindung besteht darin, daß man das Verfahren in Gegenwart eines Cu, Zn und Al enthaltenden Katalysators durchführt, der durch Kalzination und Reduktion eines Mischkristalls der Formel

$$Cu_{2,2}Zn_{2,8}(OH)_6(CO_3)_2$$

und von Aluminiumhydroxid bei Temperaturen zwischen 160 und 350°C hergestellt worden ist.

Weiterhin ein Verfahren zur Herstellung eines Katalysators, bei dem man den Mischkristall durch gemeinsame Fällung der Nitrate mit Natriumhydrogencarbonat herstellt.

Als Ausgangsstoff wird zweckmäßig ein Synthesegas verwendet, das durch partielle Oxidation von Kohlenwasserstoffen hergestellt wurde und das nach dem kombinierten $CO_2$ und $H_2S$-Wäschen nur noch CO und $H_2$ enthält und dem 1 bis 5 Vol.-% Wasserdampf zugesetzt werden.

Nach diesem Verfahren erhält man Methanol durch Umsetzung von Wasserstoff, Kohlenmonoxid und Kohlendioxid und/oder Wasserdampf und/oder Sauerstoff bei Temperaturen zwischen 200 und 350°C und Drücken von 50 bis 250 bar an einem Katalysator, der Zink, Kupfer und Aluminium in folgenden Atomprozenten enthält: 38,5% Kupfer, 48,8% Zink und 12,9% Aluminium. Zur Herstellung dieses Katalysators werden die Komponenten durch gemeinsame Fällung mit Carbonat, z. B. $NaHCO_3$ in einem leicht zersetzlichen Mischkristall überführt, d. h. im Gitter des Zinkhydroxidcarbonats sind die Zinkatome teilweise durch Kupferatome statistisch ersetzt, so daß die Komponenten bereits in der Fällung sehr fein verteilt werden. Das Aluminium wird als Hydroxid mitgefällt und optimal in der Mischkristallmatrix verteilt. Die Reduktion unter gleichzeitiger Zersetzung solcher Mischkristalle liefert dann feinverteiltes Kupfer in einer feinkristallinen Zinkoxidmatrix, in der Aluminium eingebaut ist. Das Zinkoxid ist somit aluminiumdotiert und ergibt überraschenderweise die Aktivitätssteigerung des erfindungsgemäßen Katalysators. Durch die feine Verteilung in der feinkristallinen Zinkoxidmatrix ist die Rekristallisation des Kupfers bei der erfindungsgemäßen Zusammensetzung des Katalysators minimal.

Im Vergleich zu den bekannten Verfahren liefert das Verfahren nach der Erfindung Methanol in wesentlich besserer Raumzeitausbeute. Die Lebensdauer des Katalysators ist aufgrund der geringeren Rekristallisation erhöht, und die Herstellungskosten sind durch den geringen Kupfergehalt stark gesenkt worden.

Wegen der höheren Aktivität des erfindungsgemäßen Katalysators ist der Bedarf geringer, und man kann entsprechend den Reaktorraum verkleinern. Die Wirtschaftlichkeit des Verfahrens wird durch die genannten Faktoren gerade auch bei größeren Anlagen und im kontinuierlichen Betrieb erhöht.

Neben dem Steam-Reforming-Verfahren zur Erzeugung von Synthesegas, bei dem die Ausgangsprodukte Naphtha oder Erdgas zuerst entschwefelt werden, da die Gaserzeugung an Nickelkatalysatoren erfolgt, gibt es eine weitere Möglichkeit der Synthesegaserzeugung durch partielle Oxidation von Rohölen. Bei diesem Verfahren wird z.B. schweres Heizöl mit Sauerstoff und Wasserdampf in einem Brenner verbrannt. Aufgrund der hohen Temperaturen von 1400°C entsteht ein Gasgemisch etwa folgender Zusammensetzung: 47 Vol.-Co; 48 Vol.-% $H_2$ und 5 Vol.-% $CO_2$.

Durch teilweise Konvertierung kann der gewünschte CO-Gehalt eingestellt werden. Mittels geeigneter Wäschen läßt sich dann das $H_2S$, COS und $CO_2$ bis unter 1 ppm herauswaschen, und man erhält dann ein Synthesegas aus CO und $H_2$, welches unter Zusatz von ca. 2 Vol.-% Wassersdampf und/oder ca. 0,05 bis 2 Vol.-% Sauerstoff nach dem erfindungsgemäßen Verfahren direkt für die Methanolsynthese verwendet werden kann.

Als Ausgangsstoffe des Verfahrens verwendet man vorteilhaft ein Gemisch von Kohlenmonoxid, Wasserstoff und Kohlendioxid und/oder Wasserdampf und/oder Sauerstoff, in dem in der Regel die Gase in einem Verhältnis von 0,06 bis 0,25, vorzugsweise 0,1 bis 0,2 Mol Kohlenmonoxid und von 0,01 bis 0,2, vorzugsweise 0,03 bis 0,15 Mol, Kohlendioxid und 0,01 bis 0,07, vorzugsweise 0,02 bis 0,04 Mol, Wasserdampf und 0,001 bis 0,03 Mol Sauerstoff je Mol Wasserstoff vorliegen.

Die Ausgangsstoffe können ferner auch unter den Reaktionsbedingungen sich inert verhaltende Gase wie z. B. Stickstoff, Argon und Methan bis zu etwa 5 Vol.-% insgesamt enthalten. Der Schwefel- und Chlorgehalt des Gemisches soll zweckmäßig unter 0,1 ppm liegen. Als Katalysatorvorläufer dient ein Mischkristall vom Zinkhydroxidcarbonat-Typ der Formel:

$$Cu_{2,2}Zn_{2,8}(OH)_6(CO_3)_2$$

Dieser mit Aluminium dotierte Vorläufer wird entweder direkt unter gleichzeitiger Zersetzung reduziert oder zunächst bei einer Temperatur zwischen 200 und 300°C kalziniert und dann reduziert.

Der erfindungsgemäß verwendete Katalysator wird im allgemeinen in folgender Weise hergestellt:

Man gibt eine Lösung, die die Komponenten in dem oben genannten Gewichtsverhältnis in Form ihrer Salze enthält, bei 90°C zu einer wäßrigen Lösung von Natriumbicarbonat. Die Metallsalze sind vorzugsweise Nitrate, es kommen aber auch andere wasserlösliche Salze, z. B. Acetate, in Betracht. Während der Fällung wird die Lösung gut durchmischt, damit keine großen Konzentrationsunterschiede auftreten können. Die entstehende Mischkristallfällung kann schnell röntgenographisch auf ihre Güte geprüft werden. Das Röntgenbeugungsdiagramm des Mischkristalls wird mindestens durch folgende d-Werte der Beugungslinien charakterisiert: 6,75; 3,70; 3,21; 2,90; 2,74; 2,64; 2,15; 1,94; 1,61.

Der Niederschlag wird filtriert, und das Filtergut mehrmals mit Wasser gewaschen bis im Filtrat kein Nitrat mehr nachweisbar ist. Bei 110°C wird anschließend das Filtergut getrocknet und danach gegebenenfalls noch bei 250 bis 300°C kalziniert. Zweckmäßig wird das pulverförmige Material nun in grobkörnige Form gebracht, z. B. können mit Hilfe einer Tablettiermaschine Katalysatortabletten mit den Abmessungen 3×3 mm oder auch eine gröbere Körnung hergestellt werden. Bevorzugt sind Korngrößen von 3 bis 8 Millimeter. Als Tablettierhilfsmittel wird bevorzugt Graphit in einer Menge von 1 bis 3 Gewichtsprozent bezogen auf das Kalzinat zugesetzt. Der Preßdruck wird vorteilhaft so gewählt, daß das Schüttgewicht der Tabletten zwischen 1,0 und 1,5 Kilogramm pro Liter liegt. In der Regel werden die Tabletten dann mit Wasserstoff, z. B. in einer Menge von 0,5 bis 30 Vol.-% im Gemisch mit Stickstoff, drucklos bei Temperaturen zwischen 160 und 230°C reduziert, indem man die Temperatur stufenweise steigert. Nach beendeter Reduktion liegen im wesentlichen Kupfer als Metall und die anderen Metalle oxidisch vor.

Mit dem erfindungsgemäß hergestellten Katalysator füllt man einen für Hochdruckumsetzungen geeigneten Reaktor, z. B. Schichten- oder Röhrenreaktor, und leitet das Gemisch der Ausgangsstoffe bei der Reaktionstemperatur und dem Reaktionsdruck durch den Reaktor. Bevorzugt ist eine Belastung von 5 bis 25, vorzugsweise von 10 bis 20 cbm Ausgangsgemisch, berechnet auf Normalbedingungen je Liter Katalysator und Stunde. Gegebenenfalls kann man die Reduktion des Katalysators im Reaktor und anschließend die Umsetzung des Synthesegases vornehmen. Aus dem den Reaktor verlassenden Reaktionsgemisch wird das Methanol in bekannter Weise, z. B. durch Kondensation abgetrennt.

## Beispiel 1

### A) Herstellung des Katalysators

Zur Fällung der Komponenten werden zwei Lösungen hergestellt:

Lösung 1: 1,33 kg Kupfernitrat, 2,1 kg Zinknitrat und 0,278 kg Aluminiumnitrat werden in 15 l Wasser gelöst.

Lösung 2: 2,344 kg Natriumhydrogencarbonat werden ebenfalls in 15 l Wasser gelöst.

Beide Lösungen werden getrennt auf 90°C erhitzt und dann Lösung 1 schnell (1 bis 2 Minuten) unter Rühren zu Lösung 2 gegeben. 15 Minuten wird anschließend noch gerührt und dann der Niederschlag filtriert und mit Wasser nitratfrei gewaschen. Der Filterkuchen wird bei 110°C getrocknet und anschließend bei 270°C 4 Stunden unter Stickstoffatmosphäre kalziniert. Das kalzinierte Produkt wird auf eine Korngröße unter 1 mm zerkleinert und nach Zusatz von 2 Gewichtsprozent Graphit zu 3-mm-Pillen verpreßt. Das Schüttgewicht des so hergestellten Kontaktes beträgt 1,138 g/cm³.

### B) Herstellung von Methanol

300 cm³ dieses Katalysators werden in einen Röhrenreaktor eingebaut und mit einer Mischung aus 1 Vol.-% $H_2$ und 99 Vol.-% $N_2$ drucklos reduziert. Die Temperatur wird bei der Reduktion in Zeitintervallen von je 8 Stunden von 150° auf 170° und von 170 auf 190°C erhöht und anschließend auf 230°C gesteigert.

Bei 230°C wird das Synthesegas eingeleitet, welches aus 72,6% $H_2$, 21,5% CO, 5,1% $CO_2$ und 0,8% Inerten besteht. Für die quasi adiabatische

Fahrweise wurden folgende Versuchsbedingungen gewählt:

| | |
|---|---|
| Eingangstemperatur | 230° C |
| Druck | 50 bar |
| Belastung | 10 000 $Nl/l_{Kat.} \cdot h$ |

Man erhält dann eine Raumzeitausbeute an Methanol von 1,43 kg Methanol/l Katalysator · h. Das kondensierte Rohmethanol enthält neben geringen Mengen anorganischer Verunreinigungen 96% Methanol und 3,4% Wasser. In der Fig. 1 ist die Raumzeitausbeute (A) dieses Kontaktes in Abhängigkeit von der Betriebszeit dargestellt.

Kurve 1: Metallgehalt des Katalysators in Atom-%. Cu 38,3; Zn 48,8; Al 12,9.

Als Vergleichswert ist in der Figur ein Katalysator angeführt, der eine größere Kupfermenge enthält als der erfindungsgemäße Katalysator.

Kurve 2: Metallgehalt des Katalysators in Atom-% Cu 61,6; Zn 28,1; Al 10,9.

Man erkennt hier die starke Abnahme der RZA durch verstärkte Rekristallisation des Kupfers vor allem zu Anfang des Versuches.

C) Einfluß von Wasserdampf

Der wie oben beschrieben hergestellte Katalysator wurde in einen Röhrenreaktor eingebaut und mit einem Synthesegas folgender Zusammensetzung beschickt:
74,8 Vol-% $H_2$, 21,8 Vol.-% CO, 2,7 Vol.-% $H_2O$ und 0,7 Vol.-% Inerte. Die Raumzeitausbeute beträgt ebenfalls 1,42 kg Methanol pro Liter Katalysator und Stunde. Dieser Vesuch zeigt, daß der erfindungsgemäß verwendete Katalysator eine besondere Beständigkeit gegenüber Wasserdampf aufweist.

Beispiel 2

Durch gemeinsame Fällung der Nitrate der Komponenten wird ein Katalysator hergestellt, der nach dem Trocknen, Kalzinieren und Verpressen folgende Zusammensetzung in Gewichtsprozenten besitzt: Zink 34,1%, Kupfer 27,5% und Aluminium 3,3%. Dieser Katalysator wird in einen Röhrenreaktor eingebaut und mit einem Synthesegas, bestehend aus 75,4% $H_2$, 20% CO, 4,2% $CO_2$ und 0,4% Inerte beschickt. Die Raumzeitausbeute wird in Abhängigkeit vom Druck ermittelt. Das Ergebnis ist in Fig. 2 wiedergegeben.

**Patentansprüche**

1. Verfahren zur Herstellung von Methanol durch Umsetzung eines Gasgemisches, enthaltend Kohlenmonoxid und Wasserstoff, bei Temperaturen von 200 bis 350° C und bei Drucken im Bereich von 50 bis 250 bar in Gegenwart eines Katalysators, der Zink Kupfer und Aluminium enthält, dadurch gekennzeichnet, daß man das Verfahren in Gegenwart eines Cu, Zn und Al enthaltenden Katalysators durchführt, der durch Kalzination und Reduktion eines Mischkristalls der Formel

$$Cu_{2,2}Zn_{2,8}(OH)_6(CO_3)_2$$

und von Aluminiumhydroxid bei Temperaturen zwischen 160 und 350° C hergestellt worden ist.

2. Verfahren gemäß Anspruch 1, dadurch gekennzeichnet, daß man als Ausgangsstoff ein Synthesegas verwendet, welches durch partielle Oxidation von Kohlenwasserstoffen hergestellt und das nach den kombinierten $CO_2$- und $H_2S$-Wäschen nur noch CO und $H_2$ enthält und dem 1 bis 5 Vol.-% Wasserdampf zugesetzt werden.

3. Verfahren zur Herstellung eines Katalysators für das Verfahren nach Anspruch 1, dadurch gekennzeichnet, daß man den Mischkristall durch gemeinsame Fällung der Nitrate mit Natriumhydrogencarbonat herstellt.

**Claims**

1. A process for the preparation of methanol by reacting a gaseous mixture containing carbon monoxide and hydrogen at a temperature of from 200 to 350° C and a pressure of from 50 to 250 bar over a catalyst containing zinc, copper and aluminium, wherein the process is carried out over a catalyst which contains copper, zinc and aluminium and has been prepared by calcination an and reduction of a co-crystalline material of the formula

$$Cu_{2.2}Zn_{2.8}(OH)_6(CO_3)_2,$$

and of aluminium hydroxide at from 160 to 350° C.

2. A process as claimed in Claim 1, wherein the starting material used is a synthesis gas which is prepared by partial oxidation of hydrocarbons and is subjected to a combined $CO_2$ and $H_2S$ scrubbing so that it only contains CO and $H_2$, after which from 1 to 5% by volume of steam are added.

3. A process for the preparation of a catalyst for the process as claimed in claim 1, wherein the co-crystalline material is prepared by co-precipitation of the nitrates with sodium bicarbonate.

## Revendications

1. Procédé de préparation du méthanol par réaction d'un mélange gazeux contenant de l'oxyde de carbone et de l'hydrogène, à des températures de 200 à 350°C et sous des pressions dans la gamme de 50 à 250 bars, en présence d'un catalyseur contenant du zinc, du cuivre et de l'aluminium, caractérisé en ce que le procédé est réalisé en présence d'un catalyseur au cuivre, au zinc et à l'aluminium, préparé par calcination et réduction d'un cristal mixte de la formule

$Cu_{2.2}Zn_{2.8}(OH)_6(CO_3)_2$

et d'hydroxyde d'aluminium à des températures comprises entre 160 et 350°C.

2. Procédé suivant la revendication 1, caractérisé en ce que le mélange de départ est un gaz de synthèse préparé par oxydation partielle d'hydrocarbures et qui, après extraction combinée par lavage du $CO_2$ et du $H_2S$, ne contient plus que du CO et du $H_2$ et auquel on incorpore 1 à 5% en volume de vapeur d'eau.

3. Procédé de préparation d'un catalyseur pour le procédé suivant la revendication 1, caractérisé en ce que le cristal mixte est préparé par précipitation simultanée des nitrates par l'hydrogéno-carbonate de sodium.

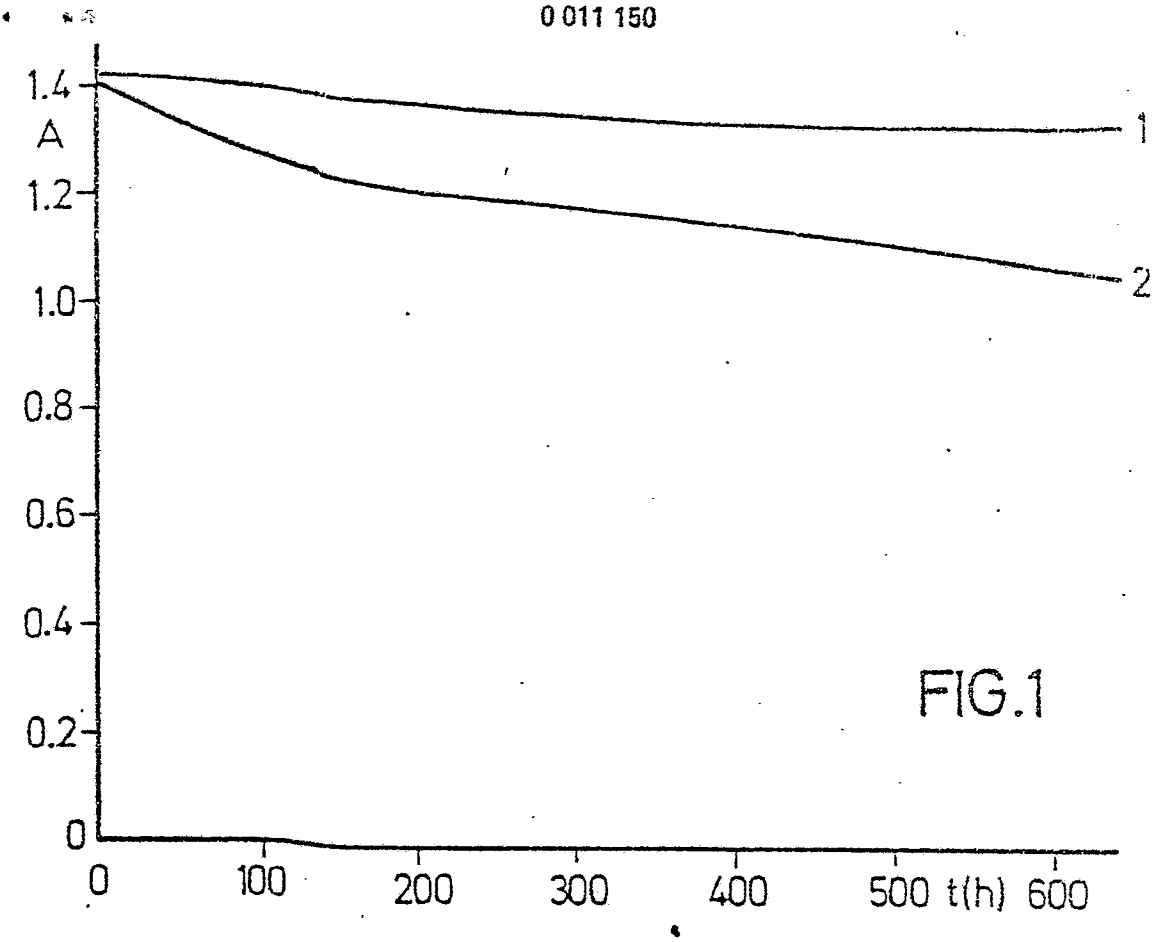
1.4
A
1.2
1.0
0.8
0.6
0.4
0.2
0
1
2
0
100
200
300
400
500 t(h) 600

FIG.1

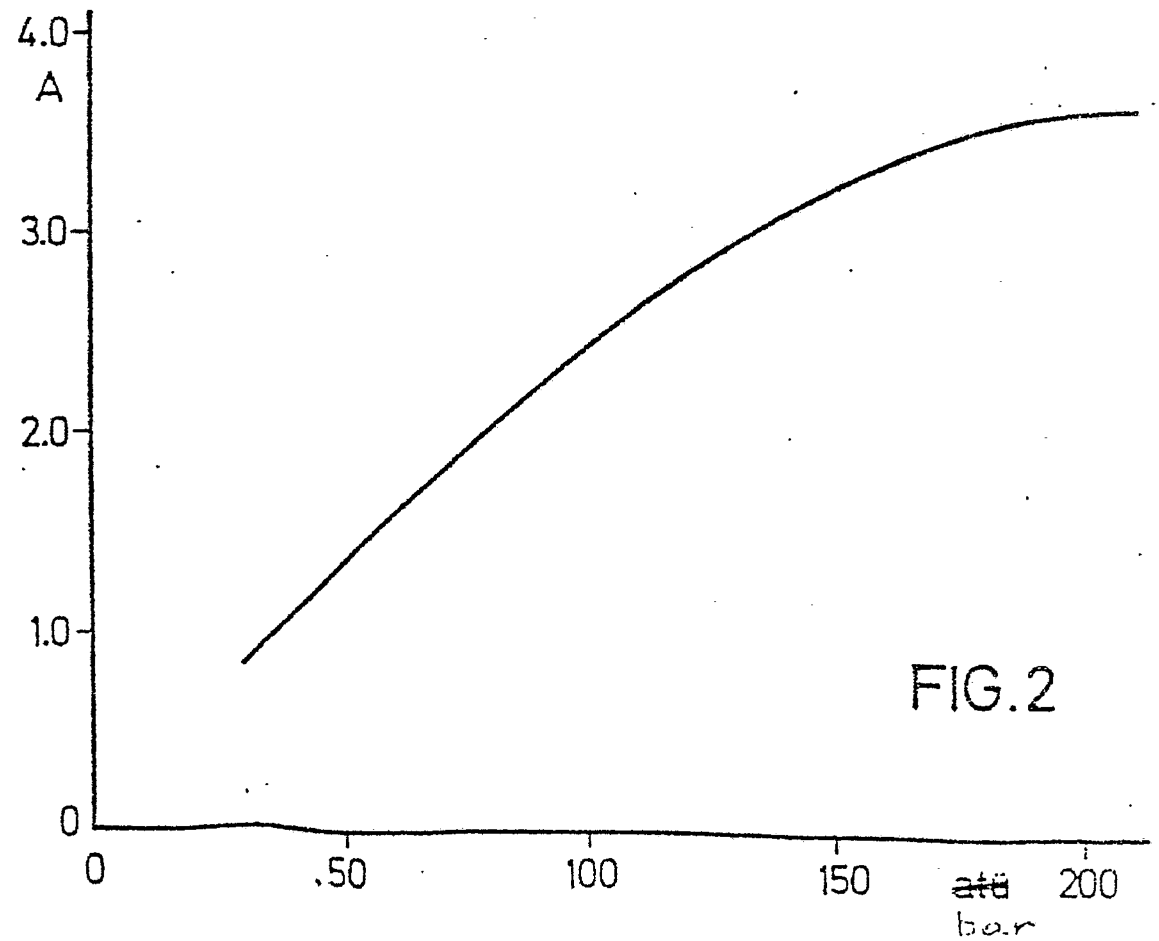
4.0
A
3.0
2.0
1.0
0
0
50
100
150
atü
bar
200

FIG.2